# EUROPEAN PATENT APPLICATION

(11) **EP 1 488 793 A1**
(43) Date of publication of application: **22.12.2004**
(21) Application number: 03715388.9
(22) Date of filing: 20.03.2003
(51) Int. Cl.: A61K 31/519, A61P 9/00, A61P 9/10, A61P 9/12, A61P 43/00

(54) **PREVENTIVES OR REMEDIES FOR DISEAES CAUSED BY eNOS EXPRESSION**

(30) Priority: 22.03.2002 JP 2002082159
(71) Applicant: Daiichi Suntory Pharma Co., Ltd., Tokyo 102-0083 (JP)
(72) Inventor: KAWASHIMA, Seinosuke, Toyonaka-shi, Osaka 560-0002 (JP); YOKOYAMA, Mitsuhiro, Kobe-shi, Hyogo 651-1112 (JP)
(74) Representative: HOFFMANN EITLE
(86) International application number: PCT/JP2003/003428
(87) International publication number: WO 2003/080063

(57) **Abstract**

The present invention provides a safe therapeutic agent with negligible adverse effects, which activates eNOS so that it optimally functions, and thereby treats or prevents diseases or pathological conditions caused by a condition where the expression product of eNOS gene does not exhibit its proper function. More specifically, the present invention relates to an agent for treating or preventing diseases or pathological conditions caused by an increase in eNOS, which contains, as an active ingredient, a compound represented by the following formula (I) or a pharmaceutically acceptable salt thereof: 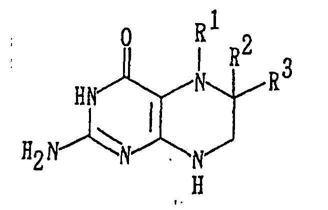 wherein each of R¹ and R² represents a hydrogen atom, or R¹ and R² together form a single bond, and when each of R¹ and R² represents a hydrogen atom, R³ represents -CH(OH)CH(OH)CH₃, -CH(OCOCH₃)CH(OCOCH₃)CH₃, -CH₃, -CH₂OH or a phenyl group, and when R¹ and R² together form a single bond, R³ represents -COCH(OH)CH₃.

## Description

### TECHNICAL FIELD

The present invention relates to a drug comprising, as an active ingredient, a compound represented by the following formula (I) or a pharmaceutically acceptable salt thereof: 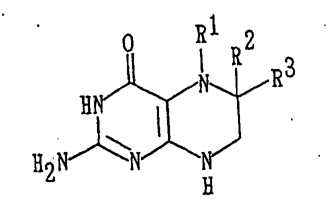 wherein each symbol represents the definition described later, which effectively prevents or ameliorates diseases or pathological conditions associated with an increase in eNOS, or pathological conditions that progress although the amount of eNOS protein and the production of NO are excessive or sufficient, wherein the prevention or amelioration is effected by suppressing or recovering from the progression of the pathological conditions, or by restoring the original function of eNOS, so as to promote the therapeutic effects against the diseases or pathological conditions.

### BACKGROUND ART

Vascular endothelium is known as a region playing an important role in the formation of vascular tonus or thrombus. In 1980, the presence of an endothelium-derived relaxing factor (EDRF) was reported for the first time. Thereafter, in 1987, it was proved that the identity of EDRF is nitrogen monoxide (NO). NO is generated when L-arginine is oxidized to L-citrulline via NG-hydroxyl-L-arginine. The reaction is catalyzed by an enzyme that is known as NO synthase (NOS). NOS is present in a wide range of organs such as vascular endothelium, nervous system, kidneys, thrombocytes, cardiac muscles, and smooth muscles. Generated NO has various actions, and it plays an important role in controlling systemic circulation.

The gene coding for NOS, has been cloned, and structural analysis has been carried out. As a result, it has been found that NOS not only has binding sites to coenzymes, such as calmodulin (CaM), flavin and NADPH, but also a binding site to (6R)-L-erythro-5,6,7,8-tetrahydrobiopterin (hereinafter referred to as "BH4") that falls within formula (I) as an active ingredient of the present invention. Moreover, it has also been suggested that BH4 actually participates in the control of the function of NOS. That is to say, NOS forms a dimer by coupling in the presence of BH4, for it to be able to produce NO (Kekkan (Blood Vessel) 2001: 24(2), 63-67). To date, Japanese Patent Laid-Open No. 10-338637 discloses that BH4 activates the function of NOS and thereby provides preventive or therapeutic effects against diseases caused by the decrease in the function of NOS. Likewise, Japanese Patent Laid-Open No. 11-246410 discloses that BH4 activates the function of NOS and thereby provides preventive or therapeutic effects against diseases accompanying the abnormality of vascular functions associated with insulin resistance. W099/43324 also discloses that BH4 activates the function of NOS to provide preventive or therapeutic effects against drug nephropathy.

Thus, the endothelium-dependent vasodilation reaction weakens in various diseases such as arteriosclerosis, hyperlipidemia, cardiac failure, hypertension and diabetes. It has become clear that NO plays a role in ameliorating these diseases by reducing the risk of the occurrence of myocardial ischemia or by reversing the decrease of exercise tolerance caused by these diseases. Moreover, it has been found that, because of its various actions, NO is deeply associated with the maintenance of angioarchitectonics, and further, with vascular remodeling. There has been suggested the possibility that NO may function as an anti-arteriosclerosis factor in an organism. For example, it has been reported that endothelial NO synthase (eNOS) gene-deficient mouse (ApoE-KO/eNOS-KO) as an animal model of atheroscrorosis does not sufficiently produce NO, and that arteriosclerosis is further accelerated in such a mouse (Kuhlencordt PJ et al., Accelerated Atherosclerosis, Aortic Aneurysm Formation, and Ischemic Heart Disease in Apolipoprotein E/Endothelial Nitric Oxide Synthase Double-Knockout Mice, Circulation 2001; 104: 448-454).

On the other hand, however, it has been found that atherosclerosis is yet further accelerated in ApoE-KO/eNOS-Tg (eNOS over-expression mouse) (American Heart Association's Scientific Sessions, November 16, 2001, Abstract No. 1312, Overexpression of Endothelial Nitric Oxide Synthase Accelerates Atherosclerotic Lesion Development in ApoE-Deficient Mice).

Moreover, it has been found that the expression of cNOS (constitutional NO synthase) in the endothelial cells of atherosclerotic blood vessels of rabbits with hereditary hyperlipidemia (Watanabe heritable hyperlipidemic rabbit WHHL) does not decrease at mRNA and protein levels, but it rather increases. It has therefore been considered that the mechanism of the decrease in the endothelium-dependent vasodilation reaction in atherosclerotic blood vessels is not caused by a reduction in NOS itself (NO to Byotai/Chiryo (NO and Pathological Conditions/Treatments), p. 36, 1995, Kawashima). Furthermore, it has been reported that the decreased endothelium-dependent vasodilation reaction in atherosclerotic blood vessels can be accompanied by an increased production of NO therein (Harrison et al., JCI 86: 2109-2116, 1990). Thus, neither the relationship between the increase in eNOS and NO nor the relationship between the increase in eNOS and pathological conditions has yet been clarified.

With regard to statin drugs for treating hyperlipidemia (Laufs U et al., Upregulation of endothelial nitric oxide synthase by HMG Co A reductase inhibitors. Circulation 1998; 97: 1129-1135), ACE inhibitors for treating hypertension and cardiac failure, AT1 antagonists (Onozato ML et al., Oxidative stress and nitric oxide synthase in rat diabetic nephropathy: Effect of ACEI and ARB. Kidney Int. 2002; 61: 186-194), Ca antagonists (Ding Y et al., Nifedipine and diltiazem but not verapamil up-regulate endothelial nitric oxide synthase expression. J Pharmacol Exp Ther. 2000; 292: 606-609), etc., it has been said that these agents increase eNOS by increasing the expression of eNOS gene or stabilizing eNOS mRNA. However, statin drugs inhibit 3-hydroxy-3-methylglutaryl-CoA (HMG-CoA), ACE inhibitors inhibit angiotensin converting enzyme, AT1 antagonists antagonize angiotensin receptor, and the Ca antagonists control the influx of Ca ions into cells through the Ca channel, respectively, in exhibiting their drug efficacy. Accordingly, the efficacy of these drugs against target diseases or pathological conditions cannot be ascribed to the mechanism of increasing eNOS through an increase in expression of eNOS gene or stabilization of eNOS mRNA.

In recent years, an attempt has been made to introduce eNOS gene into an organism, so as to recover or enhance the NO producing ability in the blood vessels. Channon et al. showed that the introduction of eNOS or nNOS gene into a rabbit resulted in a development of the vasodilatory action of NO in atherosclerotic blood vessels (Channon et al., Circulation 98: 1905-1911, 1998). Moreover, Qian et al. showed that when eNOS gene was introduced into a rabbit, which was fed on a high cholesterol diet, the expression of cell adhesion factor and the infiltration of inflammatory cells in the carotid vessels were reduced (Qian et al., Circulation 99: 2979-2982, 1999). However, some critical views on these findings point out that only short-term and localized effects are suggested, while long-term and systemic effects have not been studied. Furthermore, Kawashima et al. have found that an excessive expression of eNOS gene further aggravates atherosclerosis (American Heart Association's Scientific Sessions, November 16, 2001, Abstract No. 1312). Their findings suggest that there would be an undeniable possibility that if eNOS gene therapy was carried out to realize its therapeutic effects, long-term expression or localized excessive expression of eNOS gene might give rise to further aggravation of the disease.

### SUMMARY OF THE INVENTION

It is an object of the present invention to provide a safe therapeutic agent having negligible adverse effects, which activates the function of increased eNOS, thereby treating or preventing diseases or pathological conditions caused by a condition under which the expression product of eNOS gene does not exhibit its proper function.

Moreover, it is another object of the present invention to provide a safe therapeutic agent with negligible adverse effects, which can be used as a secondary agent *in the method for treating various diseases comprising allowing an expression of eNOS gene or promoting the expression of eNOS gene or stabilizing eNOS mRNA, by administration of a pharmaceutical agent or a gene therapy to increase eNOS*, through its ability to activate the function of the expressed or increasingly expressed eNOS.

Furthermore, it is another object of the present invention to provide a safe therapeutic agent having negligible adverse effects, which can be used as a secondary agent *in the method for treating various diseases comprising allowing an expression of eNOS gene or promoting the expression of eNOS gene*, through its ability to maintain the therapeutic effect provided by the expression or the increased expression over a long term and/or sustainably.

That is to say, the present invention is directed towards providing a safe therapeutic agent having negligible adverse effects, which effectively prevents or improves diseases or pathological conditions associated with a condition wherein eNOS does not exhibit its proper function, or pathological conditions progress when an amount of eNOS protein is excessive or sufficient, or which maintains the NO producing function of eNOS in a good condition in the treatment of diseases or pathological conditions by increasing eNOS, so that the agent improves the quality of the daily life of patients.

### BRIEF DESCRIPTION OF THE DRAWINGS

Figure 1 is a graph showing the excessive expression of eNOS in eNOS transgenic ApoE-deficient mice (ApoE-KO/eNOS-Tg);
Figure 2 is a graph showing enlarged lesion areas of the ApoE-KO/eNOS-Tg mice;
Figure 3 is a graph showing the reduction of the lesion areas of the ApoE-KO/eNOS-Tg mice by administration of BH4;
Figure 4 is a graph showing that the production of superoxide in plaque areas of the ApoE-KO/eNOS-Tg mice fed on high cholesterol diet was reduced by administration of BH4; and
Figure 5 is a graph showing that the production of NO in the ApoE-KO/eNOS-Tg mice was significantly decreased by administration of BH4.

### DISCLOSURE OF THE INVENTION

eNOS in an organism plays a role in various types of regulations by producing NO. The present inventors have found that the exhibition of the role of eNOS requires a condition where concentrations of NO and active oxygen are maintained in a good condition in an organism. They have further found that if this good condition is lost, the proper enzyme action of eNOS cannot be sufficiently exhibited, or even if it is exhibited, active oxygen increases in the organism, and the increased active oxygen aggravates the disease under treatment or causes a new disease. That is to say, the present inventors have found that an excessive amount of active oxygen is produced in a state where diseases or pathological conditions (e.g., arteriosclerosis) are aggravated, even though both the expression of eNOS gene and the production of NO are excessive or sufficient.

In order to treat and/or prevent the above diseases, the present inventors have focused their attention on the fact that NOS produces active oxygen when it is in the uncoupled form (in the state where NOS does not form a dimer). Then, the present inventors made an assumption that BH4, the most important factor involved in the coupling reaction (formation of a dimer), is a substance capable of solving the above problem. When a large amount of eNOS exists due to excessive or additional expression of eNOS gene or the stabilization of eNOS mRNA, supply of BH4 will run short, that is, the amount of BH4 becomes insufficient. It is therefore considered that, in such a condition, NOS is in the uncoupled form and generates active oxygen. Thus, the present inventors have considered that the function of eNOS can be normalized by administration of BH4.

As a result of intensive studies, the present inventors have surprisingly found that when BH4 is administered to an experimental animal model with arteriosclerosis or vascular atheroma caused by excessive expression of eNOS gene, progression of these diseases is prevented. They have, therefore, discovered that the effect of BH4 to activate the function of increased eNOS is useful for treating and preventing diseases or pathological conditions caused by an increase of eNOS, thereby accomplishing the present invention.

Accordingly, the present invention relates to a drug for preventing or treating diseases or pathological conditions caused by an increase of eNOS, which comprises, as an active ingredient, a compound represented by the following formula (I) or a pharmaceutically acceptable salt thereof: 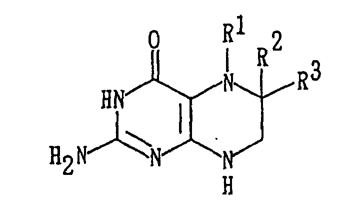 wherein each of R¹ and R² represents a hydrogen atom, or R¹ and R² together form a single bond, and when each of R¹ and R² simultaneously represents a hydrogen atom, R³ represents -CH(OH)CH(OH)CH₃, -CH(OCOCH₃)CH(OCOCH₃)CH₃, -CH₃, -CH₂OH or a phenyl group, and when R¹ and R² together form a single bond, R³ represents -COCH(OH)CH₃.

Examples of diseases or pathological conditions caused by a situation under which eNOS does not exhibit its proper function due to an increase of eNOS may include: (1) diseases or pathological conditions caused by the excessive expression of eNOS due to an abnormal expression of eNOS gene; (2) the condition that is observed when the eNOS gene is introduced from outside in a gene therapy (e.g., in the case of treatment for cardiovascular diseases or arteriosclerosis), and under such a condition eNOS is excessively expressed or the expressed eNOS does not exhibit its proper function, and thereby the expected therapeutic effects cannot be obtained or the pathological condition becomes aggravated; and (3) the condition that is observed when the eNOS increased by the administration of a statin drug, ACE inhibitor, AT1 antagonist or Ca antagonist does not exhibit the expected function, and thereby the therapeutic effects cannot be obtained or the pathological condition becomes aggravated.

The drug of the present invention is useful for therapeutically or preventively administering BH4 for the treatment of a condition in which eNOS does not exhibit its proper function due to an increase of eNOS, a condition in which eNOS gene is excessively expressed, or a case where the development of the above conditions is predicted; or for administering BH4 in combination with the introduction of the eNOS gene. When the present drug is administered in combination with the introduction of eNOS gene, the drug can be administered before, substantially at the same time of, or after the gene introduction. The administration of the present drug may be continued for a long time following gene introduction.

Conditions in which eNOS does not exhibit its proper function due to an increase of eNOS, or an excessive expression of eNOS gene, and hence requires the drug of the present invention, will take form as a cardiovascular disease, arteriosclerosis, pulmonary hypertension or the like. With regard to these diseases, in order to diagnose whether eNOS is excessively expressed or it does not function properly in an organism, the amount of active oxygen in tissues (e.g., vascular tissues) may be measured histochemically.

In order to administer the drug of the present invention in combination with agents for increasing eNOS, such as a statin drug, ACE inhibitor, AT1 antagonist or Ca antagonist, the drug of the present invention may be administered before, during or after the administration of these agents, or it may also be administered as a mixture with these agents. For example, statin drugs include atrovastatin, flavastatin, simbastatin, mevastatin, etc. The ACE inhibitors include alacepril, imidapril, enalapril, captopril, quinapril, trandopril, perindopril, ramipril, etc. The AT1 antagonists include losartan, etc., and the Ca antagonists include nifedipine, diltiazem, etc. These agents are used to inhibit vascular remodeling in diseases such as arteriosclerosis, hyperlipidemia, hypertension, heart failure and ischemic heart disease and to treat diabetic nephropathy.

The compound represented by the above formula (I) that is the active ingredient of the therapeutic agent of the present invention is known. They are known to be useful as a therapeutic agent for malignant hyperphenylalaninemia, depression, Parkinson's disease, and other diseases. Refer to, for example, Japanese Patent Laid-Open Nos. 59-25323, 59-76086, 61-277618, and 63-267781 for further information. The compound may be used as an appropriate salt. Examples of such salts may include salts of pharmacologically nontoxic acids including mineral acids such as hydrochloric acid, phosphoric acid, sulfuric acid or boric acid, and organic acids such as acetic acid, formic acid, maleic acid, fumaric acid or methansulfoniic acid.

Specific examples of the compound represented by the formula (I) that is an active ingredient of the present invention include the following compounds and pharmaceutically acceptable salts thereof:
(6R)-L-erythro-5,6,7,8-tetrahydrobiopterin (BH4), 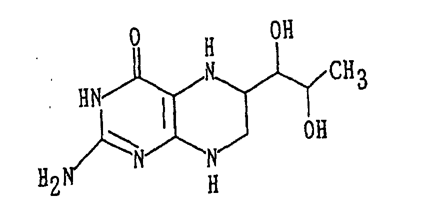
(6R, S)-5,6,7,8-tetrahydrobiopterin,
1',2'-diacetyl-5,6,7,8-tetrahydrobiopterin, 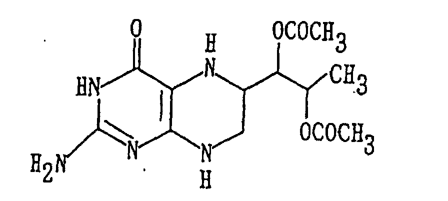
sepiapterin, 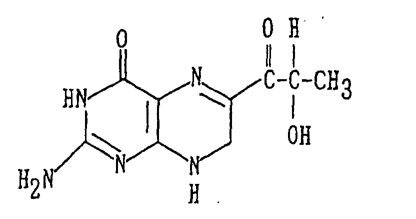
6-methyl-5,6,7,8-tetrahydropterin, 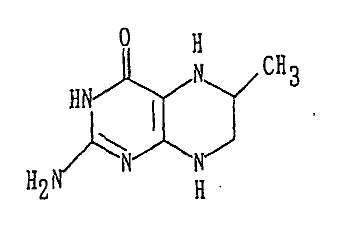
6-hydroxymethyl-5,6,7,8-tetrahydropterin, and 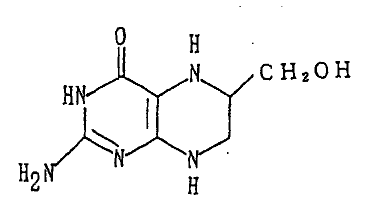
6-phenyl-5,6,7,8-tetrahydropterin.

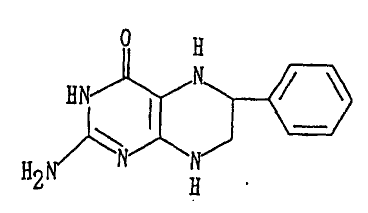

Among the above compounds, preferred compounds are 5,6,7,8-tetrahydrobiopterins and salts thereof. Further, among them, the most preferred compound is BH4 or a salt thereof.

The therapeutic agent of the present invention is produced by mixing the compound represented by the formula (I) with a carrier used for common pharmaceutical preparations by a common technique, thereby converting them into a pharmaceutical agent suitable for oral, rectal and parenteral (including intravenous and intraspinal) administration.

Carriers used for the pharmaceutical preparations can differ depending on dosage forms, but general examples of such carriers may include excipients, binders and disintegrants.

Typical examples of excipients include starch, lactose, saccharose, glucose, mannitol and cellulose, and examples of binders include polyvinylpyrrolidone, starch, saccharose, hydroxypropylcellulose and gum Arabic. Examples of disintegrants include starch, agar, gelatin powders, cellulose and CMC. However, excipients, binders and disintegrators other than the above described items may also be used, as long as they are commonly used.

In addition to the above carriers, the therapeutic agent of the present invention preferably contains an antioxidant for stabilizing the active ingredient. The antioxidant to be used can be appropriately selected from those commonly used for pharmaceutical preparations. Examples of such antioxidants may include ascorbic acid, N-acetylcysteine, L-cysteine, dl-α-tocopherol and natural tocopherol. They may be used in an amount that is necessary to stabilize the active ingredient(s) (one or more types). Generally, an antioxidant is preferably used at a weight ratio of 0.2 to 2.0:1 with respect to the active ingredient.

The drug of the present invention suitable for oral administration can be provided in the form of tablet, sublingual tablet, capsule, powder, powdered medicine, granules or parvules, or in the form of syrup, emulsion, or suspension in a non-aqueous liquid, such as potion.

In the case of granules for example, it can be produced by uniformly mixing one or more active ingredient(s) and one or more auxiliary ingredients such as the above carriers, antioxidants, granulating the mixture, and adjusting the particle size using a sieve. In the case of a tablet, it can be produced by compressing or molding one or more active ingredient(s) together with one or more auxiliary ingredients, as necessary. A capsule can be produced by uniformly mixing one or more active ingredient(s) with one or more auxiliary ingredients, as necessary, so as to obtain powders or granules, and then filling them into an appropriate capsule using a filler or the like. A preparation for rectal administration can be produced by mixing the active ingredient(s) with a common carrier such as cacao butter, so that it can be provided as a suppository. A preparation for parenteral administration can be provided by enclosing one or more active ingredient(s) in the form of dry solid into a sterilized container that has been cleaned with nitrogen. This dried solid preparation can be dispersed or dissolved in any given amount of sterile water for parenteral administration to patients.

In the production of these agents, the above described antioxidant may be preferably added to the active ingredient(s) and a common carrier. Moreover, one or more auxiliary ingredients selected from a group consisting of a buffer, flavor additive, surfactant, thickener, grease and lubricant may be further added thereto, as desired.

The amount of the active ingredient (i.e., the compound represented by the formula (I)) to be administered naturally depends on the administration route, the symptom to be treated, and the patient to be treated, but in any event, the amount will be determined by a physician.

For example, a suitable dosage is within the range between 0.1 and 50 mg/kg (body weight)/day, and a typically preferred dosage is within the range between 0.5 and 10 mg/kg (body weight)/day.

A desired dosage of the above active ingredient may be administered once per day, or it may be divided and administered two to four times per day at appropriate intervals.

The administration period may be appropriately determined by a physician, while observing the symptom to be treated by the drug of the present invention. When the drug of the present invention is administered together with drugs that are considered to increase eNOS, such as a statin drug, ACE inhibitor, AT1 antagonist or Ca antagonist, the administration period of the present drug is almost the same as the period of use of these agents. However, the administration period may also be set to be longer or shorter than the above use period, and such a period is also included in the scope of the present invention.

The active ingredient can be administered singly without mixing with other ingredients. However, in order to easily control the dosage, or for other reasons, other active ingredients can also be administered as pharmaceuticals, depending on the target diseases.

Moreover, the drug of the present invention may contain, as an auxiliary active ingredient, at least one selected from a group consisting of L-arginine, flavins (e.g., FAD, FMN, etc.) and calcium, which is a substrate, coenzyme or cofactor of NOS, together with the compound represented by the formula (I) as an active ingredient. By mixing these active ingredients, it can be expected that far superior therapeutic effects can be obtained than in the single use of the compound represented by the formula (I). The ratio of the above each ingredient contained in the drug of the present invention is not particular limited. For example, a weight ratio of at least one selected from a group consisting of L-arginine, flavins and calcium to the compound represented by the formula (I) may be 0.1 to 10, preferably 0.5 to 2.

When this mixed preparation is used for treatment, its suitable dosage is within the range of 0.1 to 50 mg/kg (body weight)/day as a total amount of active ingredients, and preferably within the range of 0.5 to 10 mg/kg (body weight)/day.

For treatment, considering age, symptoms, and so on, a physician will appropriately select which of the preparations is used; the preparation containing only the compound represented by the formula (I) as an active ingredient, or the preparation containing the above compound as well as other active ingredients.

The active ingredient used in the present invention is most preferably (6R)-L-erythro-5,6,7,8-tetrahydrobiopterin (BH4) or a salt thereof. However, analogues thereof such as (6R, S)-5,6,7,8-tetrahydrobiopterin, 1',2'-diacetyl-5,6,7,8-tetrahydrobiopterin, sepiapterin, 6-methyl-5,6,7,8-tetrahydropterin, 6-hydroxymethyl-5,6,7,8-tetrahydropterin, 6-phenyl-5,6,7,8-tetrahydropterin, or a salt thereof, may also be used. However, needless to say, BH4 that is a natural substance existing in an organism is preferable. The acute toxicity of BH4 dihydrochloride in rat is 2 g/kg (body weight) or greater for oral administration, and accordingly, almost no toxicity is found. In addition, as is clear from the description of a treatment for Parkinson's disease in Japanese Patent Laid-Open No. 59-25323, the toxicity of (6R, S)-5,6,7,8-tetrahydrobiopterin, that is, non-optically pure substance, is weak. Accordingly, this compound can also be used for the treatment in the present invention. Compounds represented by the formula (I) other than the above described compounds have almost no acute toxicity.

The present invention will be further described in the following examples. However, the examples are not intended to limit the scope of the present invention.

### EXAMPLES

### Example 1 (granule, parvule)

1 part (by weight) of polyvinylpyrrolidone (Coridon 30) was dissolved in sterile purified water. Then, 10 parts of ascorbic acid and 5 parts of L-cysteine hydrochloride were added thereto to obtain a homogenous solution. Thereafter, 10 parts of BH4 dihydrochloride was further added thereto to provide a homogenous solution.

The obtained solution was added to 59 parts of an excipient (mannitol or lactose) and 15 parts of a disintegrant [corn starch or hydroxypropylcellulose (LH-22)]. The mixture was kneaded, granulated and dried, and was then sized by a sieve.

### Example 2 (tablet)

58 parts of lactose and 15 parts of microcrystalline cellulose were mixed into the homogenous solution of the active ingredients as described in Example 1. Thereafter, 1 part of magnesium stearate was further added thereto, followed by mixing, and the mixture was tableted.

### Example 3 (capsule)

0.2% magnesium stearate was mixed as a lubricant into the granule produced in Example 1 and the granule was filled in a capsule.

### Example 4 (injection)

| | |
|---|---|
| BH4 dihydrochloride | 1.5 g |
| Ascorbic acid | 1.5 g |
| L-cysteine hydrochloride | 0.5 g |
| Mannitol | 6.5 g |

The above ingredients were dissolved in sterile purified water to prepare 100 ml of a solution. The solution was sterilized, and 1 or 2 ml each of the solution was placed in a vial or ampule, followed by freeze drying and sealing.

### Example 5 (injection)

2.0 g of BH4 dihydrochloride was dissolved in sterile purified water in the absence of oxygen, so as to prepare 100 ml of a solution. The solution was sterilized and sealed in the same manner as in Example 4.

### Example 6 (suppository)

| | |
|---|---|
| BH4 dihydrochloride | 150 parts |
| Ascorbic acid | 150 parts |
| L-cysteine hydrochloride | 50 parts |

The above ingredients were mixed to make a homogeneous powder. The powder was then dispersed in 9,950 parts of cacao butter.

### Example 7 (granule)

| | |
|---|---|
| BH4 dihydrochloride | 5 parts |
| Ascorbic acid | 5 parts |
| L-cysteine hydrochloride | 2 parts |

The above ingredients were mixed to prepare a homogenous solution.

55 parts of mannitol, 1 part of polyvinylpyrrolidone, 14 parts of hydroxypropylcellulose, and 5 parts of L-arginine or calcium were uniformly mixed, to which the above solution was added. The thus obtained mixture was kneaded, granulated, dried, and then sized using a sieve.

### Example 8 (granule)

| | |
|---|---|
| BH4 dihydrochloride | 5 parts |
| Ascorbic acid | 5 parts |
| L-cysteine hydrochloride | 5 parts |
| Mannitol | 52 parts |
| Polyvinylpyrrolidone (Coridon 30) | 1 part |
| Hydroxypropylcellulose (LH-22) | 12 parts |
| L-arginine or calcium | 10 parts |

The above ingredients were used, and granulation and sizing through a sieve classification were carried out in the same manner as in Example 7.

### Example 9 (granule)

| | |
|---|---|
| BH4 dihydrochloride | 5 parts |
| Ascorbic acid | 5 parts |
| L-cysteine hydrochloride | 2 parts |

The above ingredients were mixed to prepare a homogenous solution.

10 parts of L-arginine or calcium, 50 parts of mannitol, 1 part of polyvinylpyrrolidone (Coridon 30), and 9 parts of hydroxypropylcellulose (LH-22) were uniformly mixed, to which the above solution was added. The thus obtained mixture was kneaded, granulated, dried, and then sized using a sieve.

### Example 10 Confirmation of eNOS expression

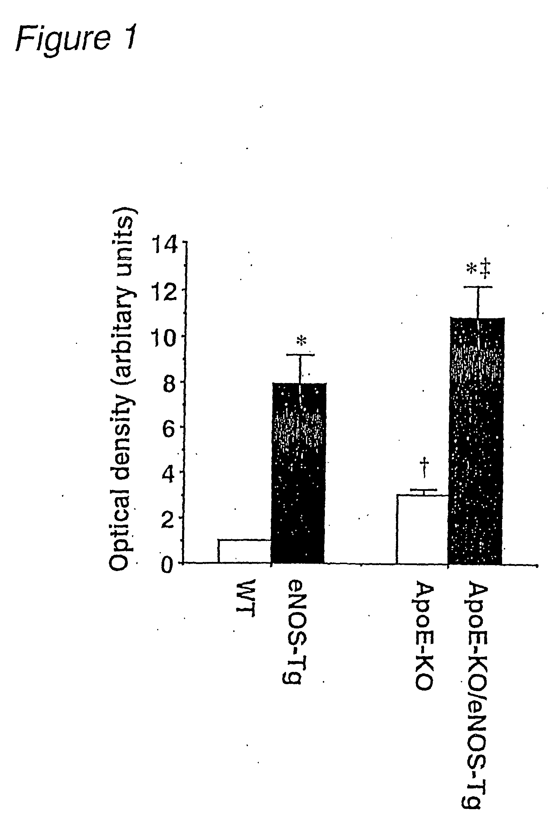

An eNOS transgenic mouse was crossed with an ApoE-deficient mouse (ApoE-KO homozygote), so as to produce an eNOS transgenic ApoE-deficient mouse (ApoE-KO/eNOS-Tg), which excessively expressed eNOS (American Heart Association's Scientific Sessions, November 16, 2001, Abstract No. 1312). The eNOS transgenic ApoE-deficient mouse could be screened by amplifying the eNOS gene according to a gene amplification method (PCR method) and measuring the amount of the amplified gene. Proteins were extracted from the aorta of a 12-week-old ApoE-KO/eNOS-Tg mouse, and the expression of eNOS was measured by immunoblotting, using a densitometer. The results are shown in Figure 1. In the figure, WT, eNOS-Tg, ApoE-KO and ApoE-KO/eNOS-Tg mean wild type, eNOS transgenic, ApoE-deficient and eNOS transgenic ApoE-deficient mouse, respectively. The graph shows the mean value ± standard deviation of six independent experiments. The asterisk denotes p < 0.01 with respect to WT, the cross denotes p < 0.05 with respect to WT, and the double cross means p < 0.05 with respect to ApoE-KO.

It was shown that the amount of eNOS proteins of the transgenic mouse is clearly greater than that of the wild type mouse, and that the transgenic mouse excessively expresses eNOS.

### Example 11 Lesion area of aortic sinus (1)

37 mice (ApoE-KO/eNOS-Tg) produced in Example 10 were ablactated at the age of 4 weeks, and then fed on high cholesterol diet (1.25% cholesterol, 7.5% cacao butter, 7.5% casein, and 0.5% sodium cholate) for 12 weeks.

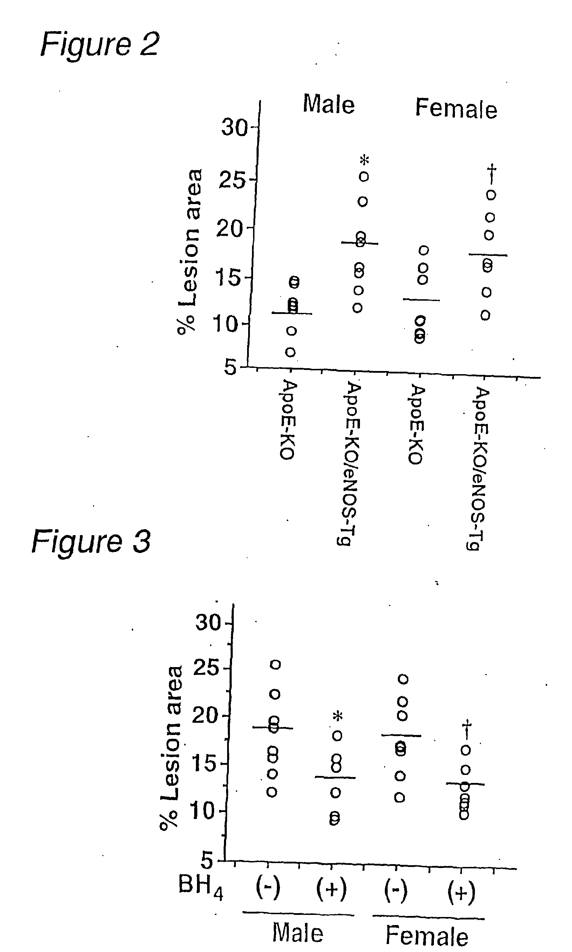

Thereafter, the 16-week-old mice were anesthetized with pentobarbital, and the aorta was perfused with a physiological saline solution containing 10 U/ml heparin. Thereafter, the aorta corresponding to the portion from the center of the left ventricle to the bifurcation portion of the iliac artery was excised and fixed with 4% paraformaldehyde overnight. Surrounding tissues were eliminated from the distal portion (from the aortic arch to the bifurcation portion of the iliac artery) of the excised aorta. The aorta was longitudinally opened and fixed on a dish coated with silicone, and then stained with Sudan III. The obtained light microscope image was analyzed using NIH 1.61 Imaging Software. The lesion formation area was expressed by lesion area/the total area of aorta. The results are shown in Figure 2. In the figure, * denotes p < 0.0001 with respect to ApoE-KO male, and the cross denotes p < 0.001 with respect to ApoE-KO female.

It was found that the lesion area increases in the eNOS transgenic ApoE-KO mice and that arteriosclerosis is thereby promoted.

### Example 12 Sclerotic Lesion area of aortic sinus (2)

6 to 8 mice (ApoE-KO/eNOS-Tg) produced in Example 10 were used per group. BH4 non-administration groups were fed on the high cholesterol diet (refer to Example 10), and BH4 administration groups were fed on the high cholesterol diet to which BH4 was added at 10 mg/kg/day. The same experiment as in Example 11 was carried out, and the aorta was excised and the same image analysis was carried out. The results are shown in Figure 3. In the figure, * denotes p < 0.05 with respect to male ApoE-KO/eNOS-Tg mice to which BH4 was not administered, and the cross denotes p < 0.01 with respect to female ApoE-KO/eNOS-Tg mice to which BH4 was not administered.

In ApoE-KO/eNOS-Tg mice to which BH4 was administered, lesion area was reduced, and arteriosclerosis was suppressed.

### Example 13 Production of superoxide

Groups were prepared, each of which consisted of 6 to 8 mice (12 weeks old, ApoE-KO/eNOS-Tg) fed on BH4 added or BH4 non-added high cholesterol diet for 8 weeks in the same manner as in Example 12. The aorta was excised from these mice, peripheral tissues were eliminated, and the remaining portion was mounted on a black silicone dish filled with PBS with pH 7.4. The sample was placed in a light-shielding box to avoid interference of external light, and thereafter, MCLA was added to the chamber to the final concentration of 20 µm/L. Thereafter, light emission generated as a result of the reaction between MCLA (2-methyl-6-(4-methoxyphenyl)-3,7-dihydroimidazol[1,2-a]pyrazin-3-one-hydrochloride) and superoxide anion was measured for 5 minutes. This measurement was repeated 3 times.

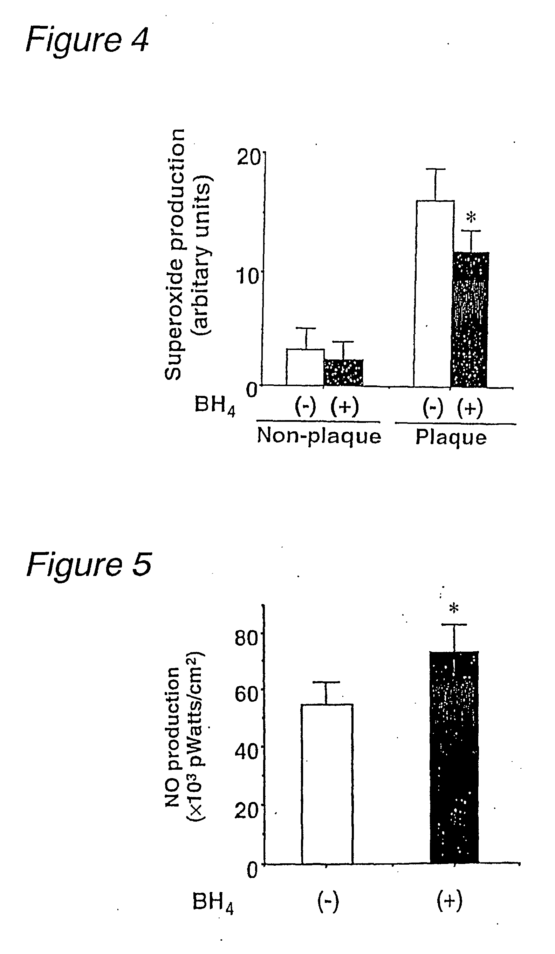

The light emission data was served in a computer, and the obtained nonlinear gray scale images were converted into pseudocolor images. The intensity of chemiluminescence due to superoxide was analyzed using WinLight 32 software. The plaque area was defined as an area that was stained with Sudan III. Approximately 10 plaque areas and 10 non-plaque areas (0.0012-0002 cm²) were randomly selected from each mouse, followed by measurement. The background level was subtracted from each chemiluminescent signal intensity. Figure 4 shows the results obtained by calculating by setting the measurement value of the wild type mouse to 1.0. The results are shown as relative values. The graph shows the mean value ± standard deviation of 6 to 10 mice of each group. In the figure, * denotes p < 0.01 with respect to ApoE-KO/eNOS-Tg mice to which BH4 was not administered.

In BH4 administration groups, in ApoE-KO/eNOS-Tg mice fed on a high cholesterol diet, the production of superoxide in their plaque area was significantly reduced, and thus, it was shown that BH4 has a superoxide inhibiting action.

### Example 14 Production of NO

The aorta containing the portion from the aortic arch to the bifurcation portion of the iliac artery was excised from each of 12-week-old mice (ApoE-KO/eNOS-Tg) fed on a high cholesterol diet for 8 weeks. Immediately thereafter, surrounding portions were eliminated in PBS with pH 7.4. The sample was mounted on a black dish filled with a phosphate buffer (pH 7.4) containing 1.5 mmol/L calcium chloride, and it was then placed in a light-shielding box to avoid interference of external light.
Diaminofluorescein-2 diacetate (DAF-2 DA) was added thereto to the final concentration of 10 µmol/L. Then, the sample was left at ambient temperature for 5 minutes, and thereafter, it was excited with 485 nm blue light. The fluorescence generated as a result of the reaction between DAF-2 DA and NO was detected, using a luminograph equipped with a band filter with a center of 532 nm, and measurement was carried out for 1 second at an intensity of wave length of 515 nm. To examine the production of NO from the aortic endothelium, the sample was left in 1 µmol/L acetylcholine solution for 5 minutes, and then the fluorescent image was measured. After completion of each experiment, in order to determine whether the measured sample was a plaque area or non-plaque area, the sample was stained with Sudan III and observed. The measurement data was input to a computer, and the non-plaque area was analyzed, using WinLight 32 software provided from NightOWL-imaging system. In order to eliminate nonspecific light emission or the reflection of collagen fibers of the aorta, the background measurement values were subtracted from the measurement after the DAF reaction. At least 10 non-plaque areas (0.0005 to 0.001 cm²) were randomly selected, and the fluorescence generated as a result of the reaction with NO was measured. The amount of the produced NO was shown as picowatt/cm². The amount of NO produced from the endothelium was obtained by subtracting the measurement value obtained under conditions where the sample was not left in an acetylcholine solution from the measurement value obtained after leaving the sample in the acetylcholine solution. The results are shown in Figure 5. The graph shows the mean value ± standard deviation of 6 independent experiments. In the figure, * denotes p < 0.01 with respect to ApoE-KO/eNOS-Tg mice to which BH4 was not administered.

In ApoE-KO/eNOS-Tg mice, the production of NO was significantly increased in the analyzed area (non-plaque area), and thus, it was shown that BH4 has an action to promote the production of NO.

### EFFECT OF THE INVENTION

The present invention provides a preventive or therapeutic agent that is effective against a condition in which eNOS does not serve its original function due to an increase of eNOS, a condition in which the eNOS gene is excessively expressed, or various diseases caused by such conditions

## Claims

1. A preventive or therapeutic agent for preventing or treating diseases or pathological conditions caused by an increase in endothelium NO synthase (eNOS), comprising, as an active ingredient, a compound represented by the following formula (I) or a pharmaceutically acceptable salt thereof: 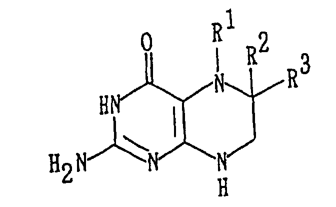 wherein each of R¹ and R² represents a hydrogen atom, or R¹ and R² together form a single bond, and when each of R¹ and R² represents a hydrogen atom, R³ represents -CH(OH)CH(OH)CH₃, -CH(OCOCH₃)CH(OCOCH₃)CH₃, -CH₃, -CH₂OH or a phenyl group, and when R¹ and R² together form a single bond, R³ represents -COCH(OH)CH₃.

2. The preventive or therapeutic agent according to claim 1, wherein the disease or pathological condition caused by an increase in eNOS is a cardiovascular disease.

3. The preventive or therapeutic agent according to claim 1, wherein the disease or pathological condition caused by an increase in eNOS is arteriosclerosis.

4. A preventive or therapeutic agent for preventing or treating the deterioration of diseases or pathological conditions caused by the increase of eNOS, comprising, as an active ingredient, the compound or a pharmaceutically acceptable salt thereof according to claim 1.

5. The preventive or therapeutic agent according to claim 4, wherein the disease or pathological condition caused by an increase in eNOS is a cardiovascular disease.

6. The preventive or therapeutic agent according to claim 4, wherein the disease or pathological condition caused by an increase in eNOS is arteriosclerosis.

7. A therapeutic agent for promoting the therapeutic effects against diseases or pathological conditions caused by an increase in eNOS, comprising, as an active ingredient, the compound or a pharmaceutically acceptable salt thereof according to claim 1.

8. The therapeutic agent according to claim 7, wherein the therapeutic effects against diseases or pathological conditions caused by an increase in eNOS are provided for cardiovascular diseases.

9. The therapeutic agent according to claim 7, wherein the therapeutic effects against diseases or pathological conditions caused by an increase in eNOS are provided for arteriosclerosis.

10. The preventive or therapeutic agent according to any one of claims 1 to 9, wherein R₃ is L-erythro-CH(OH)CH(OH)CH₃.

11. The preventive or therapeutic agent according to any one of claims 1 to 10, wherein the increase in eNOS is caused by an increase in the expression of eNOS gene.

12. The preventive or therapeutic agent according to any one of claims 1 to 11, wherein the increase of eNOS is associated with the introduction of eNOS gene.

13. The preventive or therapeutic agent according to any one of claims 1 to 11, wherein the increase of eNOS is caused by administration of a pharmaceutical agent that promotes an increase in eNOS.

14. The preventive or therapeutic agent according to any one of claims 1 to 11, further comprising a pharmaceutical agent that promotes an increase in eNOS.
